(19) Europäisches Patentamt
European Patent Office
Office européen des brevets

(11) Veröffentlichungsnummer: **0 032 659**
**B1**

(12) # EUROPÄISCHE PATENTSCHRIFT

(45) Veröffentlichungstag der Patentschrift: **19.01.83**

(51) Int. Cl.³: **C 07 C 33/20,** C 07 D 265/30, C 07 B 19/00, C 12 P 7/16, A 01 N 43/84

(21) Anmeldenummer: **81100021.5**

(22) Anmeldetag: **05.01.81**

(54) **Optisch aktive Phenylpropan-Derivate, ihre Herstellung und Verwendung zur Herstellung von Fungiziden.**

(30) Priorität: **16.01.80 DE 3001303**

(43) Veröffentlichungstag der Anmeldung: **29.07.81 Patentblatt 81/30**

(45) Bekanntmachung des Hinweises auf die Patenterteilung: **19.01.83 Patentblatt 83/3**

(84) Benannte Vertragsstaaten: **BE DE FR GB IT NL**

(56) Entgegenhaltungen:
**EP-A-0 014 999**
**DE-A-2 405 004**
**DE-A-2 446 046**
**DE-A-2 656 747**

(73) Patentinhaber: **BASF Aktiengesellschaft, Carl-Bosch-Strasse 38, D-6700 Ludwigshafen (DE)**

(72) Erfinder: **Martin, Christoph, Dr., Kolpingstrasse 6, D-6800 Mannheim (DE)**
Erfinder: **Himmele, Walter, Dr., Eichenweg 14, D-6909 Walldorf (DE)**
Erfinder: **Siegel, Hardo, Dr., Hans-Purrmann-Allee 25, D-6720 Speyer (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Optische aktive Phenylpropan-Derivate, ihre Herstellung und Verwendung zur Herstellung von Fungiziden

Die vorliegende Erfindung betrifft neue optisch aktive Phenylpropan-Derivate, ihre Herstellung und ihre Verwendung zur Herstellung fungizid wirksamer Verbindungen.

Fungizid wirksame Phenylpropan-Derivate sind bereits bekannt (vgl. DE-OS 2 656 747, DE-OS 2 750 016 und DE-OS 2 752 135). Als besonders wirksam hat sich das 1-[3-(p-tert.-Butylphenyl)-2-methyl]-cis-3,5-dimethylmorpholin I

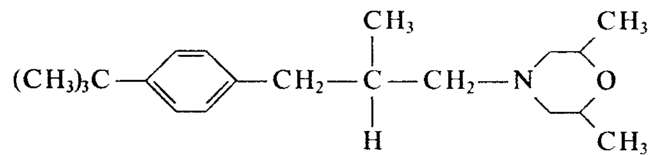

(I)

erwiesen (vgl. DE-OS 2 656 747, Anspruch 3), welches ein asymmetrisches Kohlenstoffatom besitzt. Das (—)-Enantiomere dieser Verbindung übertrifft das Racemat in seiner Wirkung. Das Racemat (I) läßt sich in bekannter Weise, z. B. durch Umsetzen mit (+)-Camphersulfonsäure in einem Verdünnungsmittel, anschließender Trennung der diastereomeren Salze durch fraktionierte Kristallisation und Umsetzung des das (—)-Enantiomere enthaltenden Salzes mit einer starken Base herstellen (vgl. die noch nicht offengelegte deutsche Patentanmeldung P 29 07 614.0 vom 27. 02. 1979). Dieses Verfahren zur Enantiomerentrennung ist jedoch aufwendig, außerdem muß das in der Mutterlauge noch enthaltene (+)-Enantiomere racemisiert werden, damit es wieder zur Enantiomerentrennung eingesetzt werden kann.

Es wurde nun gefunden, daß das (—)-Enantiomere, ausgehend von einer entsprechenden optisch aktiven Vorstufe, auf einfache Weise synthetisiert werden kann, ohne daß dabei das unerwünschte (+)-Enantiomere entsteht.

Gegenstand der Erfindung sind S-konfigurierte Phenylpropan-Derivate der Formel II

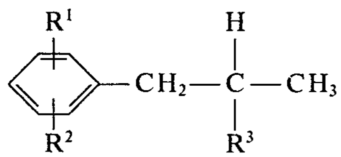

(II)

worin

$R^1$ eine Alkyl-, Aryl- oder Alkoxygruppe darstellt.
$R^2$ ein Wasserstoffatom oder identisch mit $R^1$ ist und
$R^3$ ein gegebenenfalls verestertes Carboxi, ein acetalisiertes Formyl oder ein gegebenenfalls verestertes Hydroximethyl bedeutet.

Als Bedeutungen für $R^1$ sind z. B. als Alkylgruppen solche mit 1—4 Kohlenstoffatomen, vorzugsweise die tert.-Butylgruppe, als Arylgruppe der Phenylrest und als Alkoxygruppen solche mit 1—4 Kohlenstoffatomen, vorzugsweise der tert.-Butoxirest, zu nennen.

$R^2$ ist vorzugsweise Wasserstoff.

$R^3$ ist vorzugsweise Formyl, Formyldimethylacetal, Formylethylenglykolacetal, Formylneopentylglykolacetal oder Acetoximethyl.

Gegenstand der Erfindung ist weiter ein Verfahren zur Herstellung der (S)-Verbindungen der Formel II, welches darin besteht, daß man eine Verbindung der Formel III

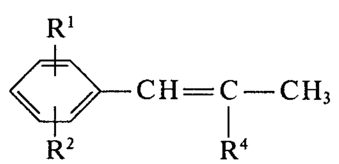

(III)

worin $R^1$ und $R^2$ dasselbe wie oben bedeuten und $R^4$ eine CHO-Gruppe oder dasselbe wie $R^3$ bedeuten, mit Hilfe von Saccharomyces cerevisiae mikrobiologisch hydriert.

Als Saccharomyces cerevisiae eignet sich Preßhefe, Bierhefe oder Backhefe.

Der Mikroorganismus kann vor der Verwendung zur Fermentation angezüchtet sein; die Anzucht erfolgt in der Regel in an sich bekannter Weise in einem wäßrigen Medium unter Zuhilfenahme der üblichen Nährstoffe. Manchmal ist es zweckmäßig, das Anzuchtmedium für die Fermentation zu verwenden, obwohl die Zusammensetzung des Fermentationsmediums wesentlich einfacher sein kann.

Die Fermentation ist ohne weitere Zusätze allein mit dem Edukt (Ausgangsmaterial) und dem

Mikroorganismus durchführbar. Es ist jedoch vorteilhaft, dem wäßrigen Medium eine assimilierbare Kohlenstoffquelle als Nährstoff zuzusetzen, vorzugsweise in einer Konzentration von 10—100 g pro Liter, beispielsweise in Form eines Zuckers, damit der Mikroorganismus möglichst lange aktiv bleibt. Der Zusatz einer Stickstoffquelle ist nicht notwendig; gegebenenfalls kann aber eine assimilierbare Stickstoffquelle zugesetzt werden, vorzugsweise in einer Menge von etwa 1—50 g pro Liter. Das Fermentationsmedium kann ferner auch anorganische Salze und andere wachstumsfördernde Substanzen, wie Vitamine, enthalten.

Der pH-Wert der Fermentation soll vorzugsweise innerhalb des Bereichs von 2 bis 10, insbesondere 3—8, liegen, ein Bereich, der zumeist ohne besondere Zusätze erreichbar ist. Die Temperatur kann in weitem Rahmen schwanken, z. B. zwischen 10°C und 40°C, wobei eine Temperatur von 20—35°C bevorzugt ist. Zur Erzielung optimaler Ausbeuten ist es vorteilhaft, daß das Edukt in der Gärbrühe in einer Konzentration von 0,1—5% vorliegt. Nach erfolgter Reduktion kann erneut Edukt zugesetzt werden. Dieses Verfahren läßt sich bis zur Inaktivierung der Mikroorganismen wiederholen.

Die Fermentationsdauer ist von dem verwendeten Mikroorganismus abhängig. Sie schwankt zwischen 5 und 200 Stunden; bei mehrmaliger Eduktzugabe kann sich die Fermentationszeit entsprechend verlängern.

Die Fermentation wird vorzugsweise aerob durchgeführt, z. B. unter Rühren, Schütteln unter Luftzutritt oder mittels einer Belüftungsvorrichtung. Vorzugsweise verwendet man einen Mikroorganismus, der sich in nicht wachsender (stationärer) Phase befindet. Neben frisch hergestellter Zellmasse können auch getrocknete oder lyophilisierte Zellen verwendet werden. Bei der mikrobiologischen Hydrierung wird eine gegebenenfalls vorhandene Aldehydgruppe ($R^4$ = CHO) zur Hydroxymethylgruppe mitreduziert.

Die glatte Hydrierung substituierter Zimtaldehyde ist überraschend, da ungesättigte Methylphenylpropan-Derivate normalerweise völlig inert gegen einen mikrobiellen Angriff sind oder aber decarboxiliert werden (vgl. Chem. Abstr. 91, 290; 35422 u.).

Die Verbindungen II lassen sich in die entsprechenden Tosylate oder Halogenderivate überführen, welche nach Umsetzung mit Aminen wie cis-Dimethylmorpholin Verbindungen der Formel IV

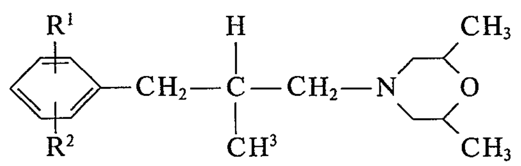

IV

worin $R^1$ und $R^2$ die angegebene Bedeutung besitzen, bilden, die hochwirksame Fungizide darstellen (vgl. DE-OS 2 656 747, DE-OS 2 752 096).

Beispiel 1

(S)-3-(p-tert.-Butylphenyl)-2-methyl-propanol-(1)

Ein sauberer, nicht sterilisierter Fermenter mit 6 l Gesamtvolumen wird folgendermaßen beschickt:

| | |
|---|---|
| Vollentsalztes Wasser | 1,8 l |
| Saccharose | 90 g |
| Preßhefe (Deutsche Hefewerke) | 200 g |
| 3-(p-tert.-Butylphenyl)-2-methyl-2-propen-1-al | 8 g in 30 ml Ethanol |
| Silicon-Antischaummittel | 2 g |

Fermentationsbedingungen sind:

| | |
|---|---|
| Temperatur | 30°C |
| Drehzahl des Rührers: | 500 U/min |
| Belüftungsrate: | 1 VVM |
| Fermentationszeit: | 52,5 h |

Die Fermentation wird nach 52,5 h abgebrochen. Die Zellmasse wird durch Zentrifugation von der Nährlösung getrennt; beide Phasen werden je 3mal mit Methylenchlorid extrahiert. Die vereinigten Extrakte werden über $Na_2SO_4$ getrocknet und im Vakuum eingedampft. Der Rückstand wird destilliert. Ausbeute an (S)-3-(p-tert.-Butylphenyl)-2-methylpropanol-1: 4,1 g (51%).

$[\alpha]_D^{20} = -7{,}54°$

Durch entsprechende Optimierung läßt sich die Ausbeute leicht steigern. Die optische Reinheit des

Produktes wurde mit Hilfe von NMR in Gegenwart von chiralen Verschiebungsreagenzien, z. B. $(Eu(hfbc)_3)$, bestimmt. Das Produkt bestand nur aus einem Enantiomeren. Durch Umwandlung des Produkts zu (S)-1-[3-(p-tert.-Butylphenyl)-2-methylpropyl]-cis-3,5-dimethylmorpholin konnte dem mikrobiellen Produkt die S-Konfiguration zugeordnet werden.

## Beispiel 2

### 2-Methylzimtalkohol-Derivate

Ein auf 30°C temperierter Rundkolben wird folgendermaßen beschickt:

300 ml vollentsalztes Wasser
50 g Saccharose
30 g Trockenhefe (Deutsche Hefewerke)

Der Ansatz wird mit 300 U/min gerührt. Nach einer Gärzeit von 15 Minuten wird 1,5 g Substrat zugegeben und weitere 24 Stunden inkubiert. Die gesamte Kulturbrühe wird anschließend 3mal mit Methylenchlorid extrahiert. Die organische Phase wird über $Na_2SO_4$ getrocknet und im Vakuum eingeengt.

Folgende Produkte wurden erhalten:

| Edukt | Produkt |
|---|---|
| 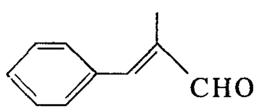 | 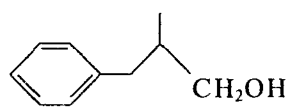 28% |
| 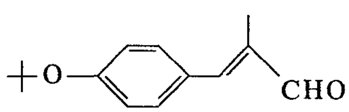 | 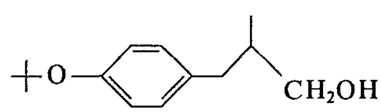 93% |
| 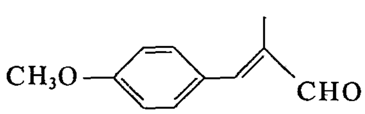 | 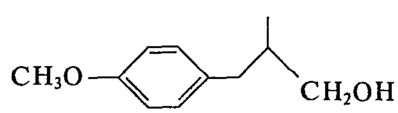 82% |
| 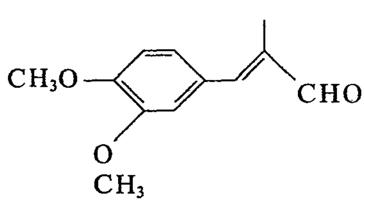 | 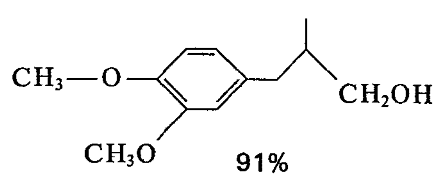 91% |

**Patentansprüche**

1. S-konfigurierte Phenylpropan-Derivate der Formel II

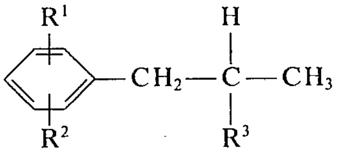

(II)

worin

$R^1$ eine Alkyl-, Aryl- oder Alkoxygruppe darstellt,
$R^2$ ein Wasserstoffatom oder identisch mit $R^1$ ist und
$R^3$ ein gegebenenfalls verestertes Carboxi, ein acetalisiertes Formyl oder ein gegebenenfalls verestertes Hydroximethyl bedeutet.

2. (S)-3-(p-tert.-Butylphenyl)-2-methyl-propanol-(1).

3. Verfahren zur Herstellung der Verbindungen gemäß Anspruch 1, dadurch gekennzeichnet, daß man eine Verbindung der Formel III

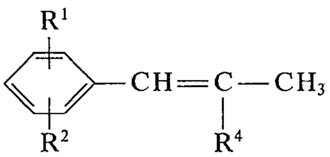

(III)

worin $R^1$ und $R^2$ dasselbe wie oben bedeuten und $R^4$ eine CHO-Gruppe oder dasselbe wie $R^3$ bedeuten, mit Hilfe von Saccharomyces cerevisiae mikrobiologisch hydriert.

4. Verwendung der Verbindungen gemäß Anspruch 1 zur Herstellung von fungizid wirksamen S-konfigurierten Verbindungen der Formel

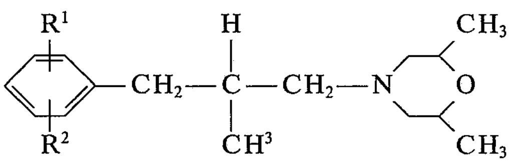

IV

worin $R^1$ und $R^2$ die in Anspruch 1 angegebene Bedeutung besitzen.

**Claims**

1. S-Configurated phenylpropane derivatives of the formula II

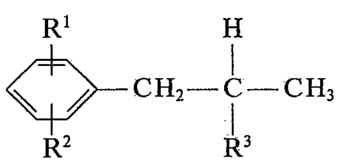

(II)

where

$R^1$ is alkyl, aryl or alkoxy,
$R^2$ is hydrogen or has the same meaning as $R^1$ and
$R^3$ is carboxyl or esterified carboxyl, acetalized formyl, hydroxymethyl or esterified hydroxymethyl.

2. (S)-3-(p-tert.-Butylphenyl)-2-methyl-propan-1-ol.

3. A process for the preparation of the compounds as claimed in claim 1, wherein a compound of the formula III

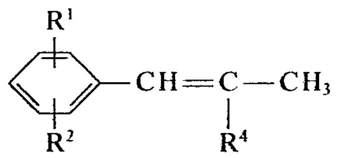

(III)

where $R^1$ and $R^2$ have the same meanings as above and $R^4$ is CHO or has the same meanings as $R^3$, is hydrogenated microbiologically with the aid of Saccharomyces cerevisiae.

4. Use of a compound as claimed in claim 1 for the preparation of a fungicidally active S-configurated compound of the formula

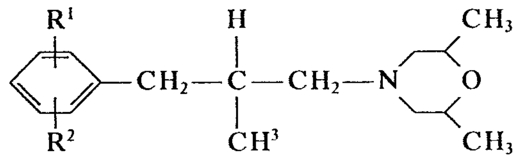

IV

where $R^1$ and $R^2$ have the meanings given in claim 1.

## Revendications

1. Dérivés du phényl-propane de configuration S de la formule II

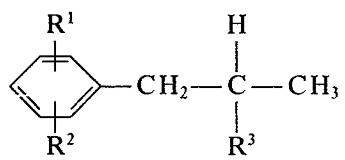

(II)

dans laquelle

$R^1$ désigne un groupe alcoyle, aryle ou alcoxy,
$R^2$ possède les mêmes significations que $R^1$ ou désigne un atome d'hydrogène et
$R^3$ représente un groupe carboxyle éventuellement estérifié ou un groupe formyle acétalisé ou un groupe hydroxyméthyle éventuellement estérifié.

2. Le (S)-3-(p-tert.-butyl-phényl)-2-méthylpropanol-(1).

3. Procédé de préparation des composés selon la revendication 1, caractérisé en ce que l'on soumet un composé de la formule III

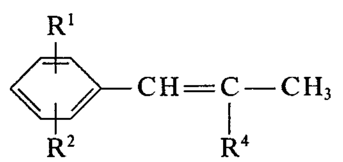

(III)

dans laquelle $R^1$ et $R^2$ possèdent les significations définies plus haut et $R^4$ possède les significations définies pour $R^3$ ou représente un groupe —CHO, à une hydrogénation microbiologique à l'aide du Saccharomyces cerevisiae.

4. Utilisation des composés selon la revendication 1 pour la préparation de composés à activité fongicide de configuration S, de la formule

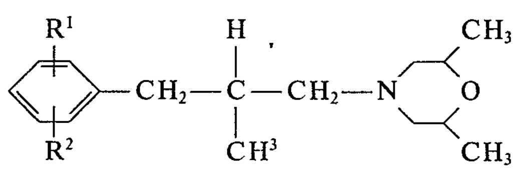

(IV)

dans laquelle $R^1$ et $R^2$ possèdent la signification définie dans la revendication 1.